(19) Europäisches Patentamt
European Patent Office
Office européen des brevets

(11) Numéro de publication : **0 204 788 B1**

(12) # FASCICULE DE BREVET EUROPÉEN

(45) Date de publication du fascicule du brevet : **30.08.89**

(51) Int. Cl.[4] : **C 07 K 7/10**, A 61 K 37/02

(21) Numéro de dépôt : **86900153.7**

(22) Date de dépôt : **10.12.85**

(86) Numéro de dépôt international : **PCT/FR 85/00356**

(87) Numéro de publication internationale : **WO/8603496 (19.06.86 Gazette 86/13)**

(54) **NOUVEAUX POLYPEPTIDES HYPOCALCEMIANTS, LEUR PREPARATION ET LES MEDICAMENTS CONTENANT CES PRINCIPES ACTIFS.**

(30) Priorité : **10.12.84 FR 8418838**
**28.12.84 FR 8419958**

(43) Date de publication de la demande : **17.12.86 Bulletin 86/51**

(45) Mention de la délivrance du brevet : **30.08.89 Bulletin 89/35**

(84) Etats contractants désignés : **AT BE CH DE FR GB IT LI LU NL SE**

(56) Documents cités :

**Néant**

(73) Titulaire : **MILHAUD, Gérard**
**7, rue des Saints Pères**
**F-75006 Paris (FR)**

(72) Inventeur : **MILHAUD, Gérard**
**7, rue des Saints Pères**
**F-75006 Paris (FR)**

**EP 0 204 788 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La présente invention a pour objet de nouveaux composés polypeptidiques répondant à la formule générale (I) caractérisés en ce qu'ils sont choisis dans le groupe formé par les produits de formule ($I_A$) :

Cys - $A_2$ - $Ser_3$ - Leu - Ser - Thr - Cys - $A_8$ - Leu - Gly -
$A_{11}$ - $A_{12}$ - $A_{13}$ - Gln - $A_{15}$ - $A_{16}$ - $A_{17}$ - Lys ($I_A$)
$A_{19}$ - $A_{20}$ - Thr - $A_{22}$ - Pro - $A_{24}$ - Thr - $A_{26}$ - $A_{27}$ -
Gly - $A_{29}$ - Gly - $A_{31}$ - Pro $NH_2$

où :

$A_2$ = Ser, Gly, Ala
$A_8$ = Val, Leu
$A_{11}$ = Lys, Thr
$A_{12}$ = Leu, Tyr
$A_{13}$ = Ser, Thr
$A_{15}$ = Glu, Asp
$A_{16}$ = Leu, Phe
$A_{17}$ = His, Asn
$A_{19}$ = Leu, Phe
$A_{20}$ = Gln, His
$A_{22}$ = Tyr, Phe
$A_{24}$ = Arg, Gln
$A_{26}$ = Asp, Asn, Ala
$A_{27}$ = Val, Thr, Ile
$A_{29}$ = Ala, Ser, Val
$A_{31}$ = Thr, Val, Ala

et les produits de formule ($I_B$) qui contiennent l'acide α-amino-subérique (Asu) dont l'α-carboxyle est fixé au groupe aminé de $A_1$ :

$(CH_2)_5$ (pont entre CO et NHCHCO)
CO-$A'_1$-$Ser_2$-Leu-Ser-Thr-NHCHCO-$A'_7$-Leu-Gly-$A'_{10}$-$A'_{11}$-$A'_{12}$-Gln-
$A'_{14}$-$A'_{15}$-$A'_{16}$-Lys-$A'_{18}$-$A'_{19}$-Thr-$A'_{21}$-Pro-$A'_{23}$-Thr-$A'_{25}$-
$A'_{26}$-Gly-$A'_{28}$-Gly-$A'_{30}$-Pro $NH_2$

où :

$A'_1$ = Ser, Gly, Ala
$A'_7$ = Val, Leu
$A'_{10}$ = Lys, Thr
$A'_{11}$ = Leu, Tyr
$A'_{12}$ = Ser, Thr
$A'_{14}$ = Glu, Asp
$A'_{15}$ = Leu, Phe
$A'_{16}$ = His, Asn
$A'_{18}$ = Leu, Phe
$A'_{19}$ = Gln, His
$A'_{21}$ = Tyr, Phe
$A'_{23}$ = Arg, Gln
$A'_{25}$ = Asp, Asn, Ala
$A'_{26}$ = Val, Thr, Ile
$A'_{28}$ = Ala, Ser, Val
$A'_{30}$ = Thr, Val, Ala

Les produits de formule ($I_B$) sont des analogues structuraux de peptides naturels, comme K. Jost et J. Rudinger les ont réalisés dans le cas des hormones neurohypophysaires (Collect. Czech. Chem. Comm., 1967, 32, 1229), des peptides donc qui répondent à des séquences d'amino acides qui diffèrent des séquences revendiquées.

La formule générale (I) qui regroupe les formules ($I_A$) et ($I_B$) peut également être représentée sous la forme suivante :

D-A'$_7$-Leu-Gly-A'$_{10}$-A'$_{11}$-A'$_{12}$-Gln-A'$_{14}$-A'$_{15}$-A'$_{16}$-Lys-A'$_{18}$-
A'$_{19}$-Thr-A'$_{21}$-Pro-A'$_{23}$-Thr-A'$_{25}$-A'$_{26}$-Gly-A'$_{28}$-Gly-Pro $NH_2$-
A'$_{30}$

dans laquelle A'$_7$, A'$_{10}$, A'$_{11}$, A'$_{12}$, A'$_{14}$, A'$_{15}$, A'$_{16}$, A'$_{18}$, A'$_{19}$, A'$_{21}$, A'$_{23}$, A'$_{25}$, A'$_{26}$, A'$_{28}$ et A'$_{30}$ ont la signification précédente et D représente :

— soit le reste

Cys- $A_2$ - $Ser_3$ - Leu - Ser - Thr - Cys -

dans lequel $A_2$ a la signification précédente

— soit le reste

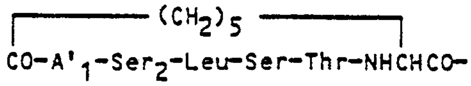

dans lequel A'$_1$ a la signification précédente.

Les polypeptides de formule (I) abaissent la calcémie et la phosphatémie ; ils inhibent la destruction osseuse, accélèrent la formation d'os nouveaux, augmentent l'absorption intestinale du calcium et diminuent la calciurie. Ils fixent le calcium à l'intérieur des cellules. Ils possèdent un effet anti-inflammatoire et antalgique majeur. Leurs indications principales sont représentées par la maladie de Paget, les ostéoporoses de diverses étiologies (commune, poro-malacique, cortisonique, post-traumatique, d'immobilisation et idiopathique), l'ostéodystrophie rénale, les fractures, les hypercalcémies, les douleurs ostéoarticulaires, en particulier celles consécutives aux métastases osseuses, la spasmophilie et la tétanie normocalcémique.

La synthèse de ces peptides peut être effectuée en utilisant la technique en phase solide seule ou en l'associant au procédé classique en milieu liquide.

La synthèse des peptides de formule ($I_A$) et celle de la séquence 10-31 des produits de formule ($I_B$) ont été effectuées de préférence selon la méthode suivante.

On utilise alors la technique en phase solide qui met en jeu la résine benzylhydrylaminée (P.G. Pietta et G.R. Marshall, Chem. Commun., 1970, 650), dont la préparation et l'utilisation ont été décrites (P. Rivaille, A. Robinson, M. Kamen et G. Milhaud, Helv., 1971, 54, 2772).

Dans un mode préférentiel de préparation des produits de formule ($I_A$), le groupe N-α-butyloxycarbonyle (t-Boc) protège la fonction α-aminée des aminoacides. Les fonctions latérales des aminoacides sont bloquées sous forme :

1) d'ester benzylique pour la chaîne carboxylique de l'acide aspartique,
2) d'éther benzylique pour l'hydroxyle des sérine, thréonine et tyrosine,
3) d'éther méthoxybenzylique pour le groupe sulfhydryle de la cystéine,
4) de dinitro-2,4-phényle pour l'imidazole de l'histidine,
5) de groupe benzoxycarbonyle pour la fonction α-aminée de la lysine.

Les différents acides aminés sont incorporés dans la chaîne peptidique à l'aide de dicyclohexylcarbodiimide (DCCI) hydroxybenzotriazole (HOBT) en faisant réagir successivement des aminoacides protégés en excès par rapport à la proline fixée en premier sur la résine. On laisse la réaction se poursuivre pendant deux heures. Les t-Boc sont éliminés par l'acide trifluoroacétique en solution dans le chlorure de méthylène ; après lavage au chlorure de méthylène, la neutralisation est effectuée par de la triéthylamine dans ce même solvant.

L'asparagine et la glutamine sont introduites sous forme de leurs esters paranitrophényliques, en solution dans le diméthylformamide (DMF) additionné de 1 % d'acide acétique. Si l'essai de contrôle du couplage à la ninhydrine est positif, on remplace l'acide acétique par de l'acide pivalique (1 %) ; si la coloration bleue persiste encore dans cet essai, on sature le DMF/1 % AcOH avec de l'urée. Si le couplage n'est pas total au bout de 48 heures de réaction, comme c'est le cas pour la glutamine en position 20, on acétyle la thréonine qui n'a pas réagi, par l'acétylimidazole dans le chlorure de méthylène.

A la fin, on élimine le groupe protecteur de l'imidazole de l'histine, alors que le peptide est encore lié à la résine, à pH 8, avec du mercapto-éthanol en solution dans le DMF. Le peptide est séparé de la résine par traitement à l'acide fluorhydrique liquide en présence d'anisole et de méthionine. Tous les groupes protecteurs sont éliminés au cours de cette opération.

Les peptides sont purifiés par filtration sur gel. Les fractions, correspondant aux pics principaux, sont rassemblées, lyophylisées, reprises dans un tampon à pH 8 et oxydées durant 24 heures par un courant d'air pour former le pont disulfure entre les deux résidus cystéiniques.

Le pic possédant l'activité biologique recherchée est purifié à nouveau sur résine échangeuse d'ions CMC 52 à l'aide d'un gradient acide d'élution. Le peptide obtenu est homogène à l'électrophorèse sur papier et à la chromatographie sur cellulose.

La séquence 10-31 des produits de formule ($I_B$) met en jeu la même résine et le même groupement N-α-butyloxycarbonyle (t-Boc) pour protéger la fonction α-aminée des aminoacides. Dans un mode d'exécution préférentiel du procédé de préparation des produits de formule ($I_B$).

Ce groupe N-α-butyloxycarbonyle (t-Boc) protège la fonction α-aminée des aminoacides. Les fonctions latérales des aminoacides sont bloquées sous forme :

1) d'ester benzylique pour la chaîne carboxylique des acides aspartique et glutamique ;

2) d'éther benzylique pour les hydroxyles des sérine, thréonine et d'éther 2,4-dichlorobenzyle pour la tyrosine ;

3) de tosyle pour le guanidinium de l'arginine et l'imidazole de l'histidine ;

4) de groupe 2-chlorobenzocarboxyle pour la fonction ε-aminée de la lysine.

Les différents acides aminés sont incorporés dans la chaîne peptidique à l'aide de dicyclohexylcarbodiimide (DCCI)-hydroxybenzotriazole (HOBT) en mélange équimoléculaire en faisant réagir successivement des aminoacides protégés en excès par rapport à la proline fixée en premier sur la résine. On laisse la réaction se poursuivre pendant deux heures. Le contrôle du couplage est effectué par le test à la ninhydrine suivant la méthode de Kaiser et Colescott. Même si ce test est négatif (pas de coloration bleue), chaque couplage est systématiquement répété deux fois. Les t-Boc sont éliminés par l'acide trifluoroacétique en solution dans le chlorure de méthylène ; après lavage au chlorure de méthylène, la neutralisation est effectuée par la diisopropyléthylamine (DIEA) dans le même solvant. L'asparagine et la glutamine sont introduites sous forme d'ester paranitrophényliques en solution dans le diméthylformamide (DMF). Si le couplage n'est pas total après une répétition (glutamine en position 19), on acétyle la thréonine qui n'a pas réagi par l'acide acétique et le DCCI.

Dans un mode préférentiel d'exécution du procédé de préparation des produits de formule ($I_B$), la synthèse de la séquence 1-9 est effectuée en milieu liquide. Le fragment

$$H-A_1-Ser-Leu-Ser-Thr-NH-\underset{}{\overset{(CH_2)_5COOR}{\overset{|}{CH}}}-CO$$

où $A_1$ a la signification ci-dessus et R = ester, après cyclisation, est condensé avec le tripeptide $A_7$-Leu-Gly. On a recours aux groupes protecteurs généralement utilisés pour la synthèse des peptides.

Le Peptide de formule ($I_B$) est obtenu alors comme suit :

Le nonapeptide protégé ci-dessus est converti en ester N-hydroxy-succinimide puis couplé avec le fragment 10-31 fixé sur la résine en milieu DMF à 30 °C. Le peptide est séparé de la résine par traitement à l'acide fluorhydrique liquide en présence de diméthylsulfure et de p-crésol (J.P. Tam, W.H. Heathand et R.B. Merrifield, J.A.C.S., 1983, 105, 6442). Tous les groupes protecteurs sont éliminés au cours de cette opération.

Les peptides sont purifiés par filtration sur gel. Les fractions, correspondant aux pics principaux, sont rassemblées, lyophylisées. Le pic possédant l'activité biologique recherchée est purifié à nouveau sur résine échangeuse d'ions CMC 52 par élution à l'aide d'un gradient de conductivité. Le peptide obtenu après lyophilisation apparaît homogène en électrophorèse sur papier et en chromatographie sur cellulose.

Les exemples suivants illustrent l'invention sans toutefois la limiter.

## Exemple 1

Obtention du peptide de formule ($II_A$) :

Cys - Ala - Ser - Leu - $Ser_5$ - Thr - Cys - Val - Leu -
$Gly_{10}$ - Lys - Leu - Ser - Gln - $Glu_{15}$ - Leu - His - ($II_A$)
Lys - Leu - $Gln_{20}$ - Thr - Tyr - Pro - Arg - $Thr_{25}$-
Asp - Val - Gly - Ala - $Gly_{30}$ - Thr - Proamide.

Les t-Boc aminoacides portant des fonctions latérales proviennent de BACHEM (Dubendorf, Suisse) ou sont préparés au laboratoire par la technique de Schnabel (E. Schnabel, Liebigs Ann. Chem., 702, 188, 1967) dans un autotitrateur. Leur pureté est déterminée par polarimétrie, F. et chromatographie sur couche mince de silice.

L'hydrolyse du peptide purifié est réalisée en tube scellé sous vide en milieu HCl 6 N (110 °C, 24 heures).

### I. Synthèse de la séquence.

a) Préparation de la résine (P: Rivaille, A. Robinson, M. Kamen et G. Milhaud, Helv., 1971, 54, 2772).

Benzyl-cétorésine.

Dans un ballon tricol de 2 l, muni d'un agitateur, on laisse gonfler pendant 2 heures, 50 g de polystyrène à 1 % de divinylbenzène Bio-rad $SX_1$ (200-400 mesh) dans 350 ml de nitrobenzène. Dans un autre ballon, on dissout 66 g de $AlCl_3$ dans 300 ml de nitrobenzène auxquels on ajoute 80 ml de chlorure de benzoyle. Le mélange est versé dans le premier ballon sous bonne agitation, maintenue pendant 2

heures à température ambiante. Si la résine gonfle trop, on ajoute du nitrobenzène (100 ml environ). On filtre, lave avec du dioxanne (3x), de l'acide acétique (3x), du méthanol (3x) et enfin, alternativement, avec du $CH_2Cl_2$ et $CH_3OH$ pour faire gonfler et contracter la résine en laissant chaque fois un temps de contact suffisant. On sèche dans un dessicateur sous vide toute la nuit (rendement 75 g).

Benzylhydrylamine résine.

Dans un ballon de 1 l muni de « Dean Stark » et d'un agitateur, on chauffe 500 g de formiate d'ammonium à 120-130 °C, pendant 1 à 2 heures. On ajoute la résine (50 g) en une seule fois et laisse la température monter à 160-165 °C, en une demi-heure, sous agitation efficace qui est maintenue pendant 6 heures. On filtre, lave à l'eau, puis on traite comme ci-dessus pour la benzylcétorésine. La résine séchée sous vide est chauffée à reflux et agitée pendant 8 heures dans HCl 6 N. Elle est ensuite filtrée et lavée précédemment. La capacité de fixation varie, selon la nature de l'acide aminé, entre 0,3 et 0,5 mMole/g.

b) Préparation du peptide II.

L'appareil utilisé permet d'agiter mécaniquement un tube en pyrex de 25 ml dont le compartiment central contient la résine (2 g). Aux extrémités du tube, 2 filtres de verre fritté sont destinés à l'introduction puis à l'élimination des réactifs (environ 15 ml).

Le couplage des acides aminés comporte les temps suivants :

Cycle 32

Couplage :

2 g de résine (capacité de fixation 0,5 mMoles/g) sont agités (10 mn) en milieu $CH_2Cl_2$, $-(CH_3)_2NCHO$ (DMF) (1 : 1, v/v) (10 ml) en présence de Boc-L-proline (2mM, 0,43 g). On ajoute DCCI (4 mMoles) et 1-hydroxybenzotriazole·(HOBT) (4 mMoles) et on reprend l'agitation pendant 2 heures. La résine est lavée successivement avec $CH_2Cl_2$ (1x, 2mn.), $CH_3OH$ abs (3x, 2mn.), $CH_2Cl_2$ (3x, 2mn). On effectue la réaction à la ninhydrine sur un échantillon de résine : elle est négative.

Libération du groupe aminé :

On traite la résine par le mélange $CF_3COOH$-$CH_2Cl_2$ (1 : 1, v/v) pendant 1 mn. ; on filtre et on répète l'opération en laissant 15 mn. au contact. On lave la résine avec $CH_2Cl_2$ (3x, 1 mn.). On élimine $CF_3COOH$ en traitant deux fois la résine par le mélange $Et_3N/CH_2Cl_2$ (12.5 : 87,5 v/v) (temps de contact 1 mn. puis 5 mn.). On lave avec $CH_2Cl_2$ (8x, 1mn). La résine-L-proline est titrée : elle contient 0,5 mMole/g de proline.

Cycle 31

Couplage :

On agite (10 mn.) la résine L-prolyle (1mMole) avec 4 mMoles de Boc-O-Benzyl-L-thréonine (1,23 g) dans $CH_2Cl_2$-DMF (1 : 1 v/v) (10 ml). On ajoute DCCI (4 mMoles) et HOBT (4 mMoles) et on reprend l'agitation (2 h). La résine est lavée successivement : $CH_2Cl_2$ (1x, 2 mn.), $CH_3OH$ abs (3x, 2 mn.), $CH_2Cl_2$ (3x, 2mn). La réaction à la ninhydrine est négative.

Libération du groupe aminé : Voir cycle 32.

Cycle 30 à 25

On utilise les procédés de couplage et de libération ci-dessus en faisant réagir pour :

Cycle 30 4 mMoles Boc-glycine (0,71 g)
Cycle 29 4 mMoles Boc-L-ala (0,76 g)
Cycle 28 4 mMoles Boc-glycine (0,71 g)
Cycle 27 4 mMoles Boc-L-valine (0,87 g)
Cycle 26 4 mMoles ester B-benzylique de l'acide Boc-L-aspartique
Cycle 25 4 mMoles Boc-O-benzyl-L-thréonine (1,23 g)

Cycle 24

Couplage :

On agite (10 mn.) la résine-peptide du cycle 25, préalablement lavée avec DMF (2x), avec 4 mMoles de Boc-N-γ-tosyl-L-arginine (1,41 g) en solution de DMF et on procède comme décrit pour le cycle 31.

Cycle 23

Couplage :

La résine peptide du cycle 24 est agitée (10 mn.) avec 4 mMoles de Boc-L-proline (0,88 g en milieu $CH_2Cl_2$ (10 ml). On ajoute DCCI/HOBT comme décrit pour le cycle 32. A la fin de la période de réaction, la réaction à la ninhydride était négative.

Libération : Voir cycle 32.

Cycles 22-21

On utilise les procédés de couplage et de libération utilisés dans le cycle 23 en faisant réagir :
Cycle 22 4 mMoles Boc-O-Benzyle-L-tyrosine (1,49 g)
Cycle 21 4 mMoles Boc-O-Benzyle-L-thréonine (1,24 g).

Cycle 20

Couplage :
La résine-peptide du cycle 21, préalablement lavée avec DMF (2x) est agitée (10 mn.) avec 4 mMoles de Boc-L-glutamine p-nitrophénylester (1,5 g) et 10 ml de DMF additionné de 1 % d'acide acétique. On ajoute DCCI (4 mMoles) HOBT (4 mMoles) et on agite pendant 48 h. On lave la résine avec DMF, $CH_2Cl_2$, $CH_3OH$, $CH_2Cl$ (2x).
Libération : Voir cycle 32.

Cycles 19 à 15

On utilise le procédé du cycle 31 en faisant réagir :
Cycle 19 4 mMoles Boc-L-leucine (1,0 g)
Cycle 18 4 mMoles Boc-ε-carbobenzyloxy-L-lysine (1,52 g)
Cycle 17 4 mMoles Boc-dinitro-2,4-phényl(im))L-histidine (1,23 g)
Cycle 16 4 mMoles Boc-L-leucine (1,0 g)
Cycle 15 4 mMoles ester-γ-benzylique de l'acide Boc-L-glutamique (1,3 g).

Cycle 14

On procède comme pour le cycle 20.

Cycle 13

On procède comme pour le cycle 31 en utilisant 4 mMoles de Boc-O-benzyl-L-sérine (1,0 g).

Cycles 12 à 9

On procède comme pour le cycle 31 en faisant réagir :
Cycle 12 : le dérivé utilisé dans le cycle 19.
Cycle 11 : le dérivé utilisé dans le cycle 18.
Cycle 10 : le dérivé utilisé dans le cycle 30.
Cycle 9 : le dérivé utilisé dans le cycle 19.
Cycle 8 : le dérivé utilisé dans le cycle 27.

Cycle 7

On utilise le procédé du cycle 31 en faisant réagir 4 mMoles d'éther-S-p-méthoxybenzylique de Boc-L-cystéine (1,13 g).

Cycles 6 à 2

Cycle 6 : Identique au cycle 31.
Cycle 5 : Identique au cycle 13.
Cycle 4 : Identique au cycle 19.
Cycle 3 : Identique au cycle 13.
Cycle 2 : Identique au cycle 29.

Cycle 1

On fait réagir dans les conditions du cycle 31 4 mMoles de l'éther S-p-méthoxybenzylique de la Boc-L-cystéine (1,13 g).

II. Elimination du groupe dinitrophényle de l'histidine

A 15 ml de diméthylformamide (DMF), on ajoute 2 ml de mercaptoéthanol et on ajuste le pH à 8 avec de la triéthylamine. On introduit ce réactif dans l'appareil et on agite 14 h. On filtre et on lave la résine alternativement avec du chlorure de méthylène (3 x), du méthanol (3 x). Après séchage sous vide, le poids

de la résine est de 2,85 g.

III. Libération du peptide de la résine et élimination des groupes protecteurs

On distille 15 ml d'acide fluorhydrique dans un mélange de 1,5 g de résine-peptide, 1,5 ml d'anisole. On agite 1 h. à 0 °C et 30 mn. à température ambiante. On chasse l'acide sous vide et on reprend le résidu dans 3 fois 5 ml d'acide acétique et on précipite par de l'éther éthylique exempt de péroxyde. On centrifuge à froid, on lave plusieurs fois le précipité à l'éther, on sèche sous vide. Le précipité est repris par de l'eau et le résidu insoluble éliminé par centrifugation. La solution lyophilisée fournit 800 mg de produit.

IV. Purification du peptide

Deux cents milligrammes de peptide brut sont placés au sommet d'une colonne (diamètre 2,5 cm, longueur 90 cm) de Biogel P6, (50-100 mesh). On élue à l'aide d'acide acétique 0,1 M et on recueille des fractions de 12 ml en suivant l'élution à l'aide d'un enregistreur 280 nm. Le peptide purifié est recueilli dans les fractions 23-28.

V. Cyclisation

Les fractions contenant le peptide purifié sont lyophilisées et le résidu est repris dans 500 ml de tampon hydrogénocarbonate d'ammonium 1M, pH 8, et soumis à un barbotage d'air à travers une plaque poreuse pendant 24 h. La solution est lyophilisée et le résidu purifié sur colonne CMC 52 Whatman par gradient d'acétate d'ammonium de mho 0,6 à mho 0,7, pH 4, avec un appareil LKB Ultrograd 11300. Après hydrolyse acide, la composition en acides aminés est la suivante, rapportée à la proline = 2 (entre parenthèses les chiffres théoriques) :

Ala (2) 2,20 ; Arg (1) 0,80 ; Asp (1) 1,04 ; Cys (2) 1,90 ; Glu (3) 3, 14 ;
Gly (3) 3,3 ; His (1) 0,85 ; Leu (5) 5,0 ; Lys (2) 1,95 ; Pro (2) 2,0 ;
Ser (3) 3,05 ; Thr (4) 4,26 ; Tyr (1) 0,90 ; Val (2) 2,2.

Exemple 2

Obtention du peptide de formule ($II_B$) :

$(CH_2)_5$

CO-Ala-Ser-Leu-Thr$_5$-NH-CH-CO-Val-Leu-Gly-Lys$_{10}$-Leu-
Ser-Gln-Glu-Leu$_{15}$-His-Lys-Leu-Gln-Thr$_{20}$-Tyr-Pro-Arg- ($II_B$)
Thr-Asp$_{25}$-Val-Gly-Ala-Gly-Thr$_{30}$-Proamide

Les t-Boc aminoacides portant des fonctions latérales proviennent de BACHEM (Dubendorf, Suisse) ou sont préparés au laboratoire par la technique de Schnabel (E. Schnabel, Liebigs Ann. Chem., 702, 188, 1967) dans un autotitrateur. Leur pureté est déterminée par polarimétrie, F. et chromatographie sur couche mince de silice.

L'hydrolyse du peptide purifié est réalisée en tube scellé sous vide en milieu HCl 6 N (110 °C, 24 heures).

1. Synthèse de la séquence 10-31 du peptide $II_B$

1-a/ Préparation de la résine (P. Rivaille, A. Robinson, M. Kamen et G. Milhaud, Helv., 1971, 54, 2772).

Benzyl-cétorésine

Dans un ballon tricol de 2 l, muni d'un agitateur, on laisse gonfler pendant 2 heures, 50 g de polystyrène à 1 % de divinylbenzène Bio-rad $SX_1$ (200-400 mesh) dans 350 ml de nitrobenzène. Dans un autre ballon, on dissout 66 g de $AlCl_3$ dans 300 ml de nitrobenzène auxquels on ajoute 80 ml de chlorure de benzoyle. Le mélange est versé dans le premier ballon sous bonne agitation, maintenue pendant 2 heures à température ambiante. Si la résine gonfle trop, on ajoute du nitrobenzène (100 ml environ). On filtre, lave avec du dioxanne (3x), de l'acide acétique (3x), du méthanol (3x) et enfin, alternativement, avec du $CH_2Cl_2$ et $CH_3OH$ pour faire gonfler et contracter la résine en laissant chaque fois un temps de contact suffisant. On sèche dans un dessicateur sous vide toute la nuit (rendement 75 g).

Benzylhydrylamine résine

Dans un ballon de 1 l muni de « Dean Stark » et d'un agitateur, on chauffe 500 g de formiate d'ammonium à 120-130 °C, pendant 1 à 2 heures. On ajoute la résine (50 g) en une seule fois et laisse la température monter à 160-165 °C, en une demi-heure, sous agitation efficace qui est maintenue pendant 6 heures. On filtre, lave à l'eau, puis on traite comme ci-dessus pour la benzylcétorésine. La résine séchée

sous vide est chauffée à reflux et agitée pendant 8 heures dans HCl 6 N. Elle est ensuite filtrée et lavée précédemment. La capacité de fixation varie, selon la nature de l'acide aminé, entre 0,3 et 0,5mMole/g.

1-b/ Préparation de la séquence 10-31 du Peptide $II_B$.

L'appareil utilisé permet d'agiter mécaniquement un tube en pyrex de 25 ml dont le compartiment central contient la résine (2 g). Aux extrémités du tube, 2 filtres de verre fritté sont destinés à l'introduction puis à l'élimination des réactifs (environ 15 ml).

Cycle 31

Couplage :

2 g de résine (capacité de fixation 0,5 mMoles/g) sont agités (10 mn) en milieu $CH_2Cl_2$, $(CH_3)_2NCHO$ (DMF) (1 : 1, v/v) (10 ml) en présence de Boc-L-proline (2mM, 0,43 g). On ajoute DCCI (4 mMoles) et 1-hydroxy-benzotriazole (HOBT) (4 mMoles) et on reprend l'agitation pendant 2 heures. La résine est lavée successivement avec $CH_2Cl_2$ (1x, 2mn.), $CH_3OH$ abs (3x, 2mn.), $CH_2Cl_2$ (3x, 2mn.). On effectue la réaction à la ninhydrine sur un échantillon de résine : elle est négative.

Libération du groupe aminé :

On traite la résine par le mélange $CF_3COOH$-$CH_2Cl_2$ (1 : 1, v/v) pendant 1 mn. ; on filtre et on répète l'opération en laissant 15 mn. au contact. On lave la résine avec $CH_2Cl_2$ (3x, 1 mn.). On élimine $CF_3COOH$ en traitant deux fois la résine par le mélange $Et_3N/CH_2Cl_2$ (12.5 : 87,5 v/v) (temps de contact 1 mn. puis 5 mn.). On lave avec $CH_2Cl_2$ (8x, 1mn.). La résine-L-proline est titrée : elle contient 0,5 mMole/g de proline.

Cycle 30

Couplage :

On agite (10 mn.) la résine L-prolyle (1mMole) avec 4 mMoles de Boc-O-Benzyl-L-thréonine (1,23 g) dans $CH_2Cl_2$-DMF (1 : 1 v/v) (10 ml). On ajoute DCCI (4 mMoles) et HOBT (4 mMoles) et on reprend l'agitation (2 h). La résine est lavée successivement : $CH_2Cl_2$ (1x, 2 mn.), $CH_3OH$ abs (3x, 2 mn.), $CH_2Cl_2$ (3x, 2mn.). La réaction à la ninhydrine est négative.

Libération du groupe aminé - Voir cycle 31.

Cycles 29 à 24

On utilise les procédés de couplage et de libération ci-dessus en faisant réagir pour :

Cycle 29 4 mMoles Boc-glycine (0,71 g)
Cycle 28 4 mMoles Boc-L-ala (0,76 g)
Cycle 27 4 mMoles Boc-glycine (0,71 g)
Cycle 26 4 mMoles Boc-L-valine (0,87 g)
Cycle 25 4 mMoles ester B-benzylique de l'acide Boc-L-aspartique
Cycle 24 4 mMoles Boc-O-benzyl-L-thréonine (1,23 g).

Cycle 23

Couplage :

On agite (10 mn.) la résine-peptide du cycle 24, préalablement lavée avec DMF (2x), avec 4 mMoles de Boc-N-γ-tosyl-L-arginine (1,41 g) en solution de DMF et on procède comme décrit pour le cycle 30.

Cycle 22

Couplage :

La résine peptide du cycle 23 est agitée (10 mn.) avec 4 mMoles de Boc-L-proline (0,88 g en milieu $CH_2Cl_2$ (10ml). On ajoute DCCI/HOBT comme décrit pour le cycle 31. A la fin de la période de réaction, la réaction à la ninhydride était négative.

Libération : Voir cycle 31.

Cycles 21-20

On utilise les procédés de couplage et de libération utilisés dans le cycle 22 en faisant réagir :

Cycle 21 4 mMoles Boc-O-Benzyle-L-tyrosine (1,49 g)
Cycle 20 4 mMoles Boc-O-Benzyle-L-thréonine (1,24 g).

Cycle 19

Couplage :

La résine-peptide du cycle 20, préalablement lavée avec DMF (2x) est agitée (10 mn.) avec 4 mMoles de Boc-L-glutamine p-nitrophénylester (1,5 g) et 10 ml de DMF additionné de 1 % d'acide acétique. On

ajoute DCCI (4 mMoles) HOBT (4 mMoles) et on agite pendant 48 h. On lave la résine avec DMF, $CH_2Cl_2$, $CH_3OH$, $CH_2Cl$ (2x).

Libération : Voir cycle 31.

Cycles 18 à 14

On utilise le procédé du cycle 30 en faisant réagir :
Cycle 18 4 mMoles Boc-L-leucine (1,0 g)
Cycle 17 4 mMoles Boc-ε-carbobenzyloxy-L-lysine (1,52 g)
Cycle 16 4 mMoles Boc-dinitro-2,4-phényl(im)L-histidine (1,23 g)
Cycle 15 4 mMoles Boc-L-leucine (1,0 g)
Cycle 14 4 mMoles ester-γ-benzylique de l'acide Boc-L-glutamique (1,3 g).

Cycle 13

On procède comme pour le cycle 19.

Cycle 12

On procède comme pour le cycle 30 en utilisant 4 mMoles de Boc-O-benzyl-L-sérine (1,0 g).

Cycles 11 et 10

On procède comme pour le cycle 30 en faisant réagir :
Cycle 11 : Le dérivé utilisé dans le cycle 18.
Cycle 10 : Le dérivé utilisé dans le cycle 17.

2. Synthèse de la séquence 1-9 du peptide $II_B$.

2-a) Séquence partielle BOC-Thr (Bzl)-Asu OMe (A).

On introduit Z-Asu Qme (11,2 g) dans OMeH-$H_2O$ (2 : 1, v/v) et on hydrogène en présence de charbon palladié (14 h.). Le catalyseur éliminé, on concentre sous vide. On ajoute du dioxanne (40 ml), puis la triéthylamine (4,2 ml) en refroidissant puis le BOC-Thr (Bzl) OSu (16 g). Après agitation (72 h.), on introduit de la N,N-diméthylamino-1,3-propane diamine, agite (4 h.), concentre au tiers. Extraction avec acétate d'éthyle (AcOEt). Lavage de AcOEt avec HCl (1 N) puis $H_2O$. Distillation sous vide. Reprise du résidu huileux dans l'éther, extraction avec $NaHCO_3$ (5 %). Réextraction avec AcOEt et lavages successifs avec $H_2O$, HCl (1 N), $H_2O$. Séchage sur sulfate de sodium anhydre. AcOEt est chassé. On obtient A sous forme de produit huileux (10 g).

2-b) Séquence partielle BOC-Ser (Bzl)-Thr (Bzl) Asu OMe.CHA (B).

A (5 g) est dissous en refroidissant dans TFA (15 ml). A température ambiante, TFA est chassé sous vide et le résidu séché sous vide. Il est dissous dans DMF (10 ml) et le pH amené à 6 à l'aide de triéthylamine (4 ml) en refroidissant. On ajoute alors HOBT (5 g) et BOC-Ser (Bzl) OSu et on agite (3 j.) à température ambiante, à pH 6. On ajoute de la N,N-diméthylamino 1,3-propanediamine, agite (1 h.), ajoute $H_2O$, extrait par AcOEt comme décrit en ci-dessus. La cyclohexylamine (CHA) est ajoutée à AcOEt séché. Distillation sous pression réduite. On obtient le produit huileux B (4 g).

2-c) Séquence partielle BOC-Ala-Ser (Bzl)-Leu-Ser (Bzl)-Thr (Bzl)-Asu OMe (C).

B (2 g) est introduit dans AcOEt en présence de HCl (1 N) ; séchage sur sulfate de sodium anhydre et concentration sous vide. Addition à froid de TFA (6 ml), agitation à température ambiante (0,5 H.), élimination du TFA sous vide, séchage du résidu sous vide. Dissolution du résidu dans DMF (2 ml), neutralisation avec triéthylamine puis introduction lente à — 40 °C dans une solution de BOC-Ala-Ser (Bzl)-Leu-NH-$NH_2$ (1,7 g) dans DMF (6 ml), à laquelle on a préalablement ajouté dioxanne (2,8 ml) en présence HCl (1 N) et nitrite d'isoamyle (0,6 ml). Ajustement du pH à 7 avec triéthylamine ; réaction à 5 °C (72 h.).

Introduction lente du mélange réactionnel à — 5 °C dans HCl 0,5 N (60 ml). Lavage du précipité par $H_2O$. Extraction par $CHCl_3$ (100 ml) ; lavages successifs avec HCl (1 N) NaCl aqueux. Elimination du $CHCl_3$. Précipitation par le mélange $CHCl_3$-n hexane (2 g).

2-d) Cyclisation Ala-Ser (Bzl)-Leu-Ser (Bzl)-Thr (Bzl) $Asu^{1,7}$-NH-$NH_2$ (D).

Dissolution de C (1,6 g) dans pyridine anhydre (15 ml). Addition de TFA-ONP (2,5 g). Agitation à 45 °C (3 h). Concentration sous vide. Précipitation par éther éthylique. Traitement par TFA comme ci-dessus. Cyclisation dans la pyridine anhydre à 50 °C pendant 5 h. Extraction par $CHCl_3$ et lavage comme décrit en ci-dessus. Concentration sous vide et précipitation par n-hexane.

Dissolution du précipité (2 g) dans DMF (5 ml) et $CH_3OH$ (25 ml). Addition de 15 ml d'hydrazine (80 %). Agitation à température ambiante (14 h.). Addition de $H_2O$. Filtration du précipité. Lavage à $H_2O$. Addition de $CH_3OH$ (50 ml) et chauffage à reflux. Précipitation de D (0,8 g).

2-e) Préparation de la séquence 1-9 : Ala-Ser (Bzl)-Leu-Ser (Bzl)-Thr (Bzl)-$Asu^{1,7}$-Val-Leu-Gly OH (E).

D (1 g) est en suspension dans DMF (4 ml), additionné de dioxanne HCl 4 N (1,7 ml) à — 5 °C. Ajout de nitrite d'isoamyle (0,3 ml) à — 10 °C sous agitation. Addition de H-Val-Leu-Gly (0,9 g) à — 5 °C, le pH étant amené à 7 par triéthylamine. Agitation en bain glacé (48 h.). Précipitation par HCl 0,5 N (150 ml) de E (1 g).

3. Préparation du peptide $II_B$.

3-a) E (500 mg) dissous dans DMF (2 ml) est additionné de HOBT (111 mg) et DCCI (150 mg). Agitation pendant 72 h. en présence du peptidyl résine 10-31 (300 mg) après avoir éliminé le t-BOC de $Lys_{10}$.

3-b) Clivage du peptide selon Tam et al. JACS (1983), 105, 6442.

Les quantités sont rapportées à 1 g de peptidyl résine.

1) On distille HF (2,5 ml) dans le mélange résine-peptide (1 g), diméthyl-sulfure (6,5 ml), p-crésol (250 mg). Agitation (1 h.) à O °C. On chasse l'acide et le diméthyl-sulfure sous vide et on reprend le résidu par 3 fois 5 ml d'AcOEt.

2) Le peptidyl résine séché, suspendu dans le diméthyl-sulfure (1 ml) est traité par HF liquide (10 ml) à 0 °C (1 h.). Après élimination du HF et du diméthyl-sulfure sous vide, la résine est lavée à l'éther (3 fois 10 ml) ; le peptide est repris à l'acide acétique et précipité à l'éther éthylique. On centrifuge à froid, on lave plusieurs fois le précipité à l'éther, on sèche sous vide. Le précipité est repris par de l'eau et le résidu insoluble éliminé par centrifugation. La solution lyophilisée fournit 500 mg de produit.

3-c) Purification du peptide.

Deux cents milligrammes de peptide brut sont placés au sommet d'une colonne (diamètre 2,5 cm, longueur 90 cm) de Biogel P6, 50-100 mesh. On élue à l'aide d'acide acétique 0,1 M et on recueille des fractions de 12 ml en suivant l'élution à l'aide d'un enregistreur 280 nm. Le peptide purifié est recueilli dans les fractions 23-28. Ces fractions sont lyophilisées et le résidu purifié sur colonne CMC 52 Whatman par gradient d'acétate d'ammonium du mho 0,6 à mho 7, pH 4, avec un appareil LKB Ultrograd 11300.

Après hydrolyse acide, la composition en acides aminés est la suivante, rapportée à la proline = 2 (entre parenthèses les chiffres théoriques) :

Ala (2) 2,1 ; Arg (1) 0,85 ; Asp (1) 1,1 ; Glu (3) 3,20 ; Gly (3) 3,2 ; His (1) 0,80 ; Leu (5) 5,2 ; Lys (2) 1,90 ; Pro (2) 2,0 ; Ser (3) 3,10 ; Thr (4) 4,20 ; Tyr (1) 0,85 ; Val (2) 2,1 ; Asu (1) 0,95.

Abréviations :

AcOEt = Acétate d'éthyle
Asu = Acide α-aminosubérique
Bzl = Benzyle
CHA = Cyclohexylamine
DCHA = Dicyclohexylamine
ONP = Ester p-nitrophénylique
OSu = Ester N-hydroxysuccinimide
Z = Benzyloxycarbonyle

Activité biologique des produits des exemples

Elle est déterminée chez le rat mâle pesant de 100 à 120 g et soumis à un jeûne de 16 heures. L'activité est exprimée en unité MRC à partir de la baisse de la calcémie, mesurée une heure après l'injection intraveineuse de peptide à tester convenablement dilué en tampon acétate de sodium 0,1 N pH 6 et 0,1 % d'albumine, par comparaison avec l'activité du « Research Standard B » convenablement dilué. L'activité biologique des peptides $II_A$ et $II_B$ est supérieure à 4 000 unités MRC par mg.

## Revendications

1. Nouveaux polypeptides répondant à la formule générale (I), caractérisés en ce qu'ils sont choisis dans le groupe formé par les produits de formule ($I_A$) :

Cys-$A_2$-$Ser_3$-Leu-Ser-Thr-Cys-$A_8$-Leu-Gly-
$A_{11}$-$A_{12}$-$A_{13}$-Gln-$A_{15}$-$A_{16}$-$A_{17}$-Lys- ($I_A$)
$A_{19}$-$A_{20}$-Thr-$A_{22}$-Pro-$A_{24}$-Thr-$A_{26}$-$A_{27}$-
Gly-$A_{29}$-Gly-$A_{31}$-Pro $NH_2$

où :

$A_2$ = Ser, Gly, Ala
$A_8$ = Val, Leu
$A_{11}$ = Lys, Thr
$A_{12}$ = Leu, Tyr
$A_{13}$ = Ser, Thr
$A_{15}$ = Glu, Asp

$A_{16}$ = Leu, Phe
$A_{17}$ = His, Asn
$A_{19}$ = Leu, Phe
$A_{20}$ = Gln, His
$A_{22}$ = Tyr, Phe
$A_{24}$ = Arg, Gln
$A_{26}$ = Asp, Asn, Ala
$A_{27}$ = Val, Thr, Ile
$A_{29}$ = Ala, Ser, Val
$A_{31}$ = Thr, Val, Ala

et les produits de formule ($I_B$) :

$$\overbrace{CO-A'_1-Ser_2-Leu-Ser-Thr-NH\underset{}{C}}^{(CH_2)_5}HCO-A'_7-Leu-Gly-A'_{10}-A'_{11}-A'_{12}-Gln-A'_{14}-A'_{15}-A'_{16}-Lys-A'_{18}-A'_{19}-Thr-A'_{21}-Pro-A'_{23}-Thr-A'_{25}-A'_{26}-Gly-A'_{28}-Gly-A'_{30}-Pro\ NH_2 \qquad (I_B)$$

où :

$A'_1$ = Ser, Gly, Ala
$A'_7$ = Val, Leu
$A'_{10}$ = Lys, Thr
$A'_{11}$ = Leu, Tyr
$A'_{12}$ = Ser, Thr
$A'_{14}$ = Glu, Asp
$A'_{15}$ = Leu, Phe
$A'_{16}$ = His, Asn
$A'_{18}$ = Leu, Phe
$A'_{19}$ = Gln, His
$A'_{21}$ = Tyr, Phe
$A'_{23}$ = Arg, Gln
$A'_{25}$ = Asp, Asn, Ala
$A'_{26}$ = Val, Thr, Ile
$A'_{28}$ = Ala, Ser, Val
$A'_{30}$ = Thr, Val, Ala

2. Nouveaux polypeptides répondant à la formule ($I_A$) telle que définie à la revendication 1 dans laquelle le symbole $A_8$ représente une valine.

3. Nouveaux polypeptides répondant à la formule ($I_B$) telle que définie à la revendication 1 dans laquelle le symbole $A'_7$ représente une valine.

4. Le produit de formule ($II_A$) :

$$Cys-Ala-Ser-Leu-Ser_5-Thr-Cys-Val-Leu-Gly_{10}-Lys-Leu-Ser-Gln-Glu_{15}-Leu-His-Lys-Leu-Gln_{20}-Thr-Tyr-Pro-Arg-Thr_{25}-Asp-Val-Gly-Ala-Gly_{30}-Thr-Proamide. \qquad (II_A)$$

5. Le produit de formule ($II_B$) :

$$\overbrace{CO-Ala-Ser-Leu-Thr_5-NH-C}^{(CH_2)_5}H-CO-Val-Leu-Gly-Lys_{10}-Leu-Ser-Gln-Glu-Leu_{15}-His-Lys-Leu-Gln-Thr_{20}-Tyr-Pro-Arg-Thr-Asp_{25}-Val-Gly-Ala-Gly-Thr_{30}-Proamide \qquad (II_B)$$

6. Procédé de préparation des nouveaux polypeptides spécifiés dans les revendications 1 à 5, caractérisé par le fait de soumettre des acides aminés, des peptides ou leurs combinaisons à des réactions de condensation dans l'ordre de la séquence des acides aminés de la formule I et par le fait que lors de la préparation des produits de formule ($I_B$) on associe la technique en phase solide pour l'obtention de la séquence 10-31 et la technique classique pour l'obtention de la séquence 1-9.

7. A titre de médicament, les polypeptides mentionnés dans les revendications 1 à 5.

8. Les préparations pharmaceutiques contenant comme principe actif un ou plusieurs polypeptides mentionnés dans la revendication 1 à 5, ainsi que les véhicules adéquats destinés à l'administration parentérale, orale sous forme de liposome et nasale sous forme de pulvérisation.

## Claims

1. New polypeptides corresponding to the general formula (I), characterized in that they are chosen from the group formed by the products of formula ($I_A$) :

Cys-$A_2$-$Ser_3$-Leu-Ser-Thr-Cys-$A_8$-Leu-Gly-
$A_{11}$-$A_{12}$-$A_{13}$-Gln-$A_{15}$-$A_{16}$-$A_{17}$-Lys-
$A_{19}$-$A_{20}$-Thr-$A_{22}$-Pro-$A_{24}$-Thr-$A_{26}$-$A_{27}$-
Gly-$A_{29}$-Gly-$A_{31}$-Pro $NH_2$ ($I_A$)

where :

$A_2$ = Ser, Gly, Ala
$A_8$ = Val, Leu
$A_{11}$ = Lys, Thr
$A_{12}$ = Leu, Tyr
$A_{13}$ = Ser, Thr
$A_{15}$ = Glu, Asp
$A_{16}$ = Leu, Phe
$A_{17}$ = His, Asn
$A_{19}$ = Leu, Phe
$A_{20}$ = Gln, His
$A_{22}$ = Tyr, Phe
$A_{24}$ = Arg, Gln
$A_{26}$ = Asp, Asn, Ala
$A_{27}$ = Val, Thr, Ile
$A_{29}$ = Ala, Ser, Val
$A_{31}$ = Thr, Val, Ala

and the products of formula ($I_B$) :

┌──────$(CH_2)_5$──────┐
CO-$A'_1$-$Ser_2$-Leu-Ser-Thr-NHCHCO-$A'_7$-Leu-Gly-$A'_{10}$-
$A'_{11}$-$A'_{12}$-Gln-$A'_{14}$-$A'_{15}$-$A'_{16}$-Lys-$A'_{18}$-$A'_{19}$-
Thr-$A'_{21}$-Pro-$A'_{23}$-Thr-$A'_{25}$-$A'_{26}$-Gly-$A'_{28}$-Gly-
$A'_{30}$-Pro $NH_2$ ($I_B$)

where :

$A'_1$ = Ser, Gly, Ala
$A'_7$ = Val, Leu
$A'_{10}$ = Lys, Thr
$A'_{11}$ = Leu, Tyr
$A'_{12}$ = Ser, Thr
$A'_{14}$ = Glu, Asp
$A'_{15}$ = Leu, Phe
$A'_{16}$ = His, Asn
$A'_{18}$ = Leu, Phe
$A'_{19}$ = Gln, His
$A'_{21}$ = Tyr, Phe
$A'_{23}$ = Arg, Gln
$A'_{25}$ = Asp, Asn, Ala
$A'_{26}$ = Val, Thr, Ile
$A'_{28}$ = Ala, Ser, Val
$A'_{30}$ = Thr, Val, Ala

2. New polypeptides corresponding to the formula ($I_A$) as defined in claim 1 in which the symbol $A_8$ represents a valine.

3. New polypeptides corresponding to the formula ($I_B$) as defined in claim 1 in which the symbol $A'_7$ represents a valine.

4. The product of formula ($II_A$) :

Cys-Ala-Ser-Leu-$Ser_5$-Thr-Cys-Val-Leu-
$Gly_{10}$-Lys-Leu-Ser-Gln-$Glu_{15}$-Leu-His-
Lys-Leu-$Gln_{20}$-Thr-Tyr-Pro-Arg-$Thr_{25}$-
Asp-Val-Gly-Ala-$Gly_{30}$-Thr-Proamide. ($II_A$)

5. The product of formula ($II_B$) :

$(CH_2)_5$

CO-Ala-Ser-Leu-$Thr_5$-NH-CH-CO-Val-Leu-Gly-$Lys_{10}$-Leu-
Ser-Gln-Glu-$Leu_{15}$-His-Lys-Leu-Gln-$Thr_{20}$-Tyr-Pro-Arg-
Thr-$Asp_{25}$-Val-Gly-Ala-Gly-$Thr_{30}$-Proamide  ($II_B$)

6. Preparation process for new polypeptides specified in claims 1 to 5, characterized by the fact that amino acids, peptides or their combinations are submitted to condensation reactions in the order of sequence of the amino acids of formula (I) and by the fact that during the preparation of the products of formula ($I_B$) the solid phase technique for obtaining the 10-31 sequence and the standard technique for obtaining the 1-9 sequence are combined.

7. As medicaments, the polypeptides mentioned in claims 1 to 5.

8. The pharmaceutical preparations containing as active principle one or more polypeptides mentioned in claims 1 to 5, as well as appropriate vehicles intended for parenteral administration, oral administration in the form of liposome and nasal administration in the form of atomization.

## Patentansprüche

1. Neue Polypeptide der allgemeinen Formel (I), dadurch gekennzeichnet, daß sie ausgewählt sind aus der Gruppe der Produkte der Formel ($I_A$)

Cys-$A_2$-$Ser_3$-Leu-Ser-Thr-Cys-$A_8$-Leu-Gly-
$A_{11}$-$A_{12}$-$A_{13}$-Gln-$A_{15}$-$A_{16}$-$A_{17}$-Lys-
$A_{19}$-$A_{20}$-Thr-$A_{22}$-Pro-$A_{24}$-Thr-$A_{26}$-$A_{27}$-
Gly-$A_{29}$-Gly-$A_{31}$-Pro $NH_2$  ($I_A$)

worin :

$A_2$ = Ser, Gly, Ala
$A_8$ = Val, Leu
$A_{11}$ = Lys, Thr
$A_{12}$ = Leu, Tyr
$A_{13}$ = Ser, Thr
$A_{15}$ = Glu, Asp
$A_{16}$ = Leu, Phe
$A_{17}$ = His, Asn
$A_{19}$ = Leu, Phe
$A_{20}$ = Gln, His
$A_{22}$ = Tyr, Phe
$A_{24}$ = Arg, Gln
$A_{26}$ = Asp, Asn, Ala
$A_{27}$ = Val, Thr, Ile
$A_{29}$ = Ala, Ser, Val
$A_{31}$ = Thr, Val, Ala

und der Produkte der Formel ($I_B$)

$(CH_2)_5$

CO-$A'_1$-$Ser_2$-Leu-Ser-Thr-NHCHCO-$A'_7$-Leu-Gly-$A'_{10}$-
$A'_{11}$-$A'_{12}$-Gln-$A'_{14}$-$A'_{15}$-$A'_{16}$-Lys-$A'_{18}$-$A'_{19}$-
Thr-$A'_{21}$-Pro-$A'_{23}$-Thr-$A'_{25}$-$A'_{26}$-Gly-$A'_{28}$-Gly-
$A'_{30}$-Pro $NH_2$  ($I_B$)

worin :

$A'_1$ = Ser, Gly, Ala
$A'_7$ = Val, Leu
$A''_{10}$ = Lys, Thr
$A'_{11}$ = Leu, Tyr
$A'_{12}$ = Ser, Thr
$A'_{14}$ = Glu, Asp
$A'_{15}$ = Leu, Phe
$A'_{16}$ = His, Asn
$A'_{18}$ = Leu, Phe

$A'_{19}$ = Gln, His
$A'_{21}$ = Tyr, Phe
$A'_{23}$ = Arg, Gln
$A'_{25}$ = Asp, Asn, Ala
$A'_{26}$ = Val, Thr, Ile
$A'_{28}$ = Ala, Ser, Val
$A'_{30}$ = Thr, Val, Ala

2. Neue Polypeptide der Formel ($I_A$), wie in Anspruch 1 definiert, in der das Symbol $A_8$ für Valin steht.

3. Neue Polypeptide der Formel ($I_B$), wie in Anspruch 1 definiert, in der das Symbol $A'_7$ für Valin steht.

4. Die Produkte der Formel ($II_A$)

Cys-Ala-Ser-Leu-$Ser_5$-Thr-Cys-Val-Leu-
$Gly_{10}$-Lys-Leu-Ser-Gln-$Glu_{15}$-Leu-His-
Lys-Leu-$Gln_{20}$-Thr-Tyr-Pro-Arg-$Thr_{25}$-
Asp-Val-Gly-Ala-$Gly_{30}$-Thr-Proamid. ($II_A$)

5. Die Produkte der Formel ($II_B$)

```
┌──────────(CH2)5──────────┐
CO-Ala-Ser-Leu-Thr5-NH-CH-CO-Val-Leu-Gly-Lys10-Leu-
Ser-Gln-Glu-Leu15-His-Lys-Leu-Gln-Thr20-Tyr-Pro-Arg-
Thr-Asp25-Val-Gly-Ala-Gly-Thr30-Proamide
```
($II_B$)

6. Verfahren zur Herstellung neuer Polypeptide, wie in den Ansprüchen 1 bis 5 spezifiziert, dadurch gekennzeichnet, daß man Aminosäuren, Peptide oder deren Kombinationen in der Reihenfolge der Sequenz der Aminosäuren der Formel I Kondensationsreaktionen unterzieht, und daß man bei der Herstellung der Produkte der Formel ($I_B$) die Technik in der festen Phase für die Gewinnung der 10-31-Sequenz und die klassische Technik für die Gewinnung der 1-9-Sequenz miteinander verbindet.

7. Als Arzneimittel die Polypeptide der Ansprüche 1 bis 5.

8. Pharmazeutische Zusammensetzungen, enthaltend als Wirkstoff ein oder mehrere Polypeptide gemäß Anspruch 1 bis 5 sowie geeignete Träger für die parenterale oder orale Verabreichung in Form von Liposomen und die nasale Verabreichung in Form von Zerstäubungspräparaten.